# EUROPEAN PATENT SPECIFICATION

(11) **EP 1 389 964 B1**
(45) Date of publication and mention of the grant of the patent: **17.05.2006**
(21) Application number: 02739422.0
(22) Date of filing: 28.05.2002
(51) Int. Cl.: A61B 17/88, A61B 17/068, B25B 23/06

(54) **INSERTION DEVICE FOR BONE FIXATION ELEMENTS**
EINSETZVORRICHTUNG FÜR KNOCHENFIXIERUNGSELEMENTE
APPAREIL D'INSERTION D' L MENTS DE FIXATION

(30) Priority: 30.05.2001 US 866841; 05.11.2001 US 330977 P
(43) Date of publication of application: 25.02.2004
(73) Proprietor: Chen, Mike C., King of Prussia, PA 19406 (US); Zwirnmann, Ralph, Roslyn, PA 19001-1520 (US); Synthes AG Chur, 7002 Chur (CH)
(72) Inventor: Chen, Mike C., Ling of Prussia, PA 19406 (US); Zwirnmann, Ralph, Roslyn, PA 19001-1520 (US)
(74) Representative: Lusuardi, Werther
(86) International application number: PCT/US2002/016656
(87) International publication number: WO 2002/096310

(56) References cited:
- EP-A- 0 834 281
- EP-A- 1 090 591
- FR-A- 2 682 587
- FR-A- 2 777 443
- US-A- 4 478 112
- US-A- 5 458 608
- US-A- 5 893 856

## Description

The present invention relates to a device for the storage and dispensing of osteosynthetic fixation elements, and in particular to a device for attaching fixation elements to bone.

US-A-5458 608 is considered to represent the closest prior art, and discloses a device for the present purpose comprising a longitudinal tubular member holding a single fastener carrier element movable under the action of a piston.

In the surgical treatment of fractures in the maxillofacial area, as well as fractures of the foot and hand, a trend toward preferring ever-smaller implants can clearly be noted. The reason for this is the generally increased understanding of the biomechanical bases of osteosynthesis. In the field of treating maxillofacial fractures, more attention can be paid to the cosmetic results of osteosynthesis, thanks to the miniaturization of implants. In the field of hand surgery, restrictions on movement in the area of the fingers can be avoided. In this regard, smaller osteosynthetic implants in the fingers can be placed under the tendons. In the case of an implant with a large cross-section, the tendons need no longer be extended to their full length.

The dimensions of some smaller implants (screws, plates and tacks) are in the range of about 0.8 mm to about 2.0 mm. Problems in the area of packaging, storage and manipulation during surgery arise due to this miniaturization. Handling in the operating room, particularly in the maxillofacial area, has proved difficult. Depending on the degree of severity of the fracture or correction, up to 40 bone fixation elements, such as tacks or screws, may be required. These screws must be taken individually by the operating room nurse from a so-called screw rack, checked for length, placed on a screwdriver and given to the surgeon. The surgeon must, in turn, insert them through the osteosynthesis plate into pre-drilled screw holes. During the transfer of the screw and the attempted insertion of the screw, it often falls off the screwdriver, into the wound or onto the operating room floor. The attempt to find a lost screw is often excessively time-consuming, given their dimensions and extends the time spent in surgery. The frequent loss of screws in the operating room, and during packing and sterilization, causes unnecessary costs for the hospital.

An additional problem in dealing with mini-screws arises during their implantation. After the surgeon has selected the osteosynthesis plate proper for the fracture in question, a plate is positioned over the fracture. A hole is then drilled for the screw (0.5-1.5 mm diameter) through one of the plate holes. After drilling, the screw is received from an operating room nurse and screwed into the bone through the plate. Commonly problems arise in finding the core drill hole in the bone, since the bone surface is covered with blood or soft tissue and the plate can slip on the smooth bone surface.

In addition, problems arise in controlling the amount of force applied during the insertion of the screw or tack. For example, if a surgeon is required to insert a screw or tack with manual force, the manual force could be transmitted to the surrounding bone, which could bend thin and/or flexible bone in young patients.

Also, problems such as surgical gloves tearing or hand pinching can arise if the insertion device has parts that move externally during the firing of the device.

Thus, a need exists for an insertion device that minimizes the manual force exertion required and to minimize the gross forces applied to the surrounding bone during insertion.

To achieve the above object the present invention relates to a device for attaching fixation elements to bone as in claim 1. Said device comprises a longitudinal member having a channel extending therein, a piston and a plurality of spacers positionable within the channel and moveable by means of the piston. Each spacer has a tip portion at a distal end configured and dimensioned for holding a fixation element.

In a special embodiment, the spacer has a cavity at a proximal end configured and dimensioned for receiving a tip portion of an adjacent spacer. The spacer can have a frustoconical portion. The fixation element is held to the tip portion by a friction fit and the spacer has a shoulder at a base of the tip portion configured for contacting a proximal end of an adjacent spacer when such spacers are in abutting relationship. In one preferred embodiment, the spacers are stackable such that a plurality of spacers are positionable in abutting relationship and the spacers can be axially alignable within the channel.

The piston can include a frustoconical tip at a distal end and the tip can be received in the proximal end of the spacer. In one embodiment, a tab extends radially outward from the piston and is configured to be movable by a human finger to move the piston with respect to the longitudinal member. A spring engages a slot in the longitudinal member for locating the piston at a plurality of preselected locations with respect to the longitudinal member.

In a further embodiment, the channel has a front opening at the distal end of the longitudinal member and the spacers can travel through the opening. A handle is connected to the proximal end of the longitudinal member.

The invention is described in more detail in the following description when read with reference to the accompanying drawings which illustrate one embodiment of the invention. In the drawings:
Fig. 1 is a perspective view of a fixation element for use with the insertion device according to the present invention;
Fig. 2 is a side view of a fixation element spacer;
Fig. 3 is a perspective view of an embodiment of an insertion device;
Fig. 4 is a cross-sectional view of the embodiment of Fig. 3.

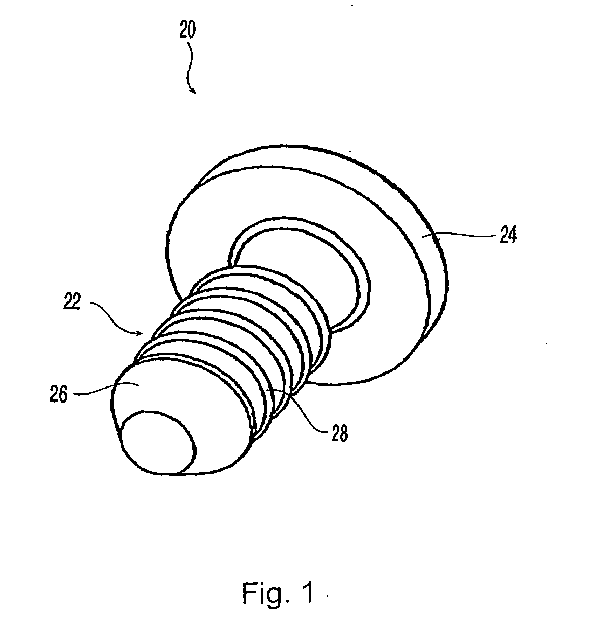

Referring to Fig. 1, one fixation element compatible with insertion device 130 comprises a tack 20 having a shaft 22 integral with a head 24 at a proximal end thereof. The distal end of shaft 22 has a conical nose 26 to facilitate the insertion of tack 20 into bone tissue. A plurality of circular ribs 28 extend radially from the exterior of shaft 22 to prevent the removal of the tack from the bone tissue after it has been inserted. Head 24 has an outer diameter greater than the diameter of shaft 22 and contacts or rests against the bone or bone plate when the tack is inserted into bone tissue. In the preferred embodiment, the tack is made from a resorbable material so that it remains in the bone tissue temporarily and is absorbed by the body. In alternate embodiments, tack 20 can have numerously different configurations and dimensions. Also, different types of fixation elements altogether can be used with insertion device 130. For example, biocompatible screws, nails, anchors, rivets, or other similar implants can also be inserted using insertion device 130.

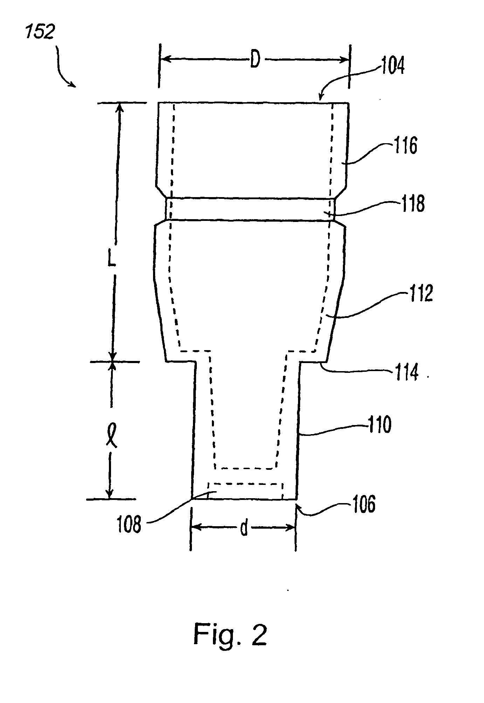

The tacks 20 are attached to spacers 152 and are aligned coaxially within the central channel 138 of device 130. In a preferred embodiment, spacer 152 is hollow and has an exterior contour and an interior contour, and the interior contour is generally configured and dimensioned to conform to the exterior contour so that a plurality of spacers are easily stacked. As best seen in Fig. 2, each spacer 152 has a circular cross section which tapers from a hollow proximal end 104 to a distal end 106 having a socket 108 for receiving a tack 20. Tack 20 is held in socket 108 by an interference or friction fit. Each spacer can accommodate at least one fixation element at the distal end, and a plurality of spacers can be inserted into central channel 138 of insertion device 130, each with a tack attached to the distal end. The spacers are aligned coaxially within central channel 138 such that each spacer 152 proximal end 104 is aligned to receive a distal end 106 of an adjacent spacer so that the spacers can be axially stacked in abutting relation within channel 138. Each spacer 152 comprises a cylindrical nose or tip portion 110 toward distal end 106 and has a diameter d. Tip portion 110 extends an axial distance I from an angled mid-section portion 112. A shoulder 114 is formed at the intersection of tip portion 110 and mid-section portion 112. Back portion 116 extends from mid-section portion 112 and includes a slot 118 extending around the outer periphery. Back portion 116 is preferably substantially cylindrical having a diameter D and mid-section portion 112 is preferably substantially frusto-conical. Back portion 116 diameter D is larger than tip portion 110 diameter d so that tip portion 110 of a spacer 152 can be accommodated within the interior of back portion 116. In a preferred embodiment, shoulder 114 extends radially beyond the perimeter of tip portion 110 and is configured and dimensioned to rest against or abut the proximal end 104 of back portion 116. Mid-section portion 112 and back portion 116 extend an axial distance L from shoulder 114 to proximal end 104, and distance L is preferably greater than distance I. In this way, when a fixation element or tack 20 is attached to a tip portion of a first spacer and then inserted into the interior of a proximal end of a second spacer, there is sufficient space within the interior of the second spacer to accommodate the tip portion of the first spacer and a fixation element attached thereto.

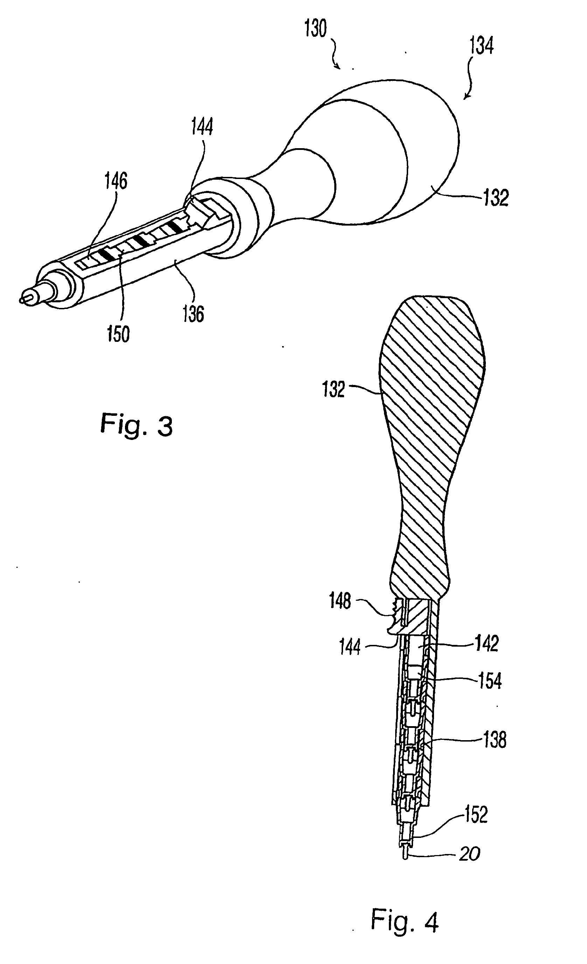

Referring to Figs. 3 and 4, an embodiment of an insertion device 130 includes a handle 132 at the proximal end 134 and an elongate applicator extension 136 extends from handle 132 in the distal direction. Applicator extension 136 has a central channel 138 for receiving a plurality of fixation elements or tacks 20 and a piston 142 is axially aligned with channel 138 and movable therein to advance the tacks in the distal direction. Piston 142 is positioned at the proximal end of channel 138 and a tab 144 extends laterally from one side of piston 142 and through a longitudinal slot 146 in extension 136. Tab 144 includes a knurled or textured portion 148 for engaging a person's finger to allow for manual advancement of piston 142 within channel 138. In a preferred embodiment, slot 146 includes angled portions 150 and a spring (not shown) is attached to tab 144 to engage angled portions 150 of slot 146 and prevent piston 142 from moving in a proximal direction. Channel 138 forms a fixation element supply chamber, and the fixation elements are attached to spacers 152 and are aligned coaxially within channel 138 to be inserted into bone tissue one at a time as described above. The configuration and design of the spacers 152 used with device 130 are the same as spacer 152 shown in Fig. 2. Also, the fixation elements are held at the distal end of each spacer by an interference or friction fit. In a preferred embodiment, channel 138 is configured to accommodate about four spacers, however the invention is also applicable to different size insertion devices with various channel lengths to accommodate more or less spacers or fixation elements as desired.

In operation of insertion device 130, the distal end of piston 142 interfaces engages the proximal end of the last or proximal most spacer aligned within channel 138 to force the spacer in the distal direction and advance all of the aligned spacers in the channel. In a preferred embodiment, piston 142 exterior substantially corresponds to the interior of the mid-section and back portion of spacer 152. For example, piston 142 is preferably generally cylindrical with a tapered portion 154 at the distal end and tapered portion 154 is angled to generally correspond to the interior of mid-section portion of spacer 152.

## Claims

1. A device for attaching fixation elements to bone, having a longitudinal member (136) extending along a longitudinal axis extending from a handle (132) at its proximal end (134) to a distal end having a channel (138) extending therein and a manually advancable piston (142) located centrally with respect to said channel (138), said device further comprising
A) a plurality of spacers (152) positionable within the channel (138) and movable by means of said piston (142) axially with respect to said channel (138), whereby
B) each spacer (152) comprises a body extending from a distal body end (106) to a proximal body end (104) and
C) the distal body end (106) is adapted to receive at least a portion of a fixation element, whereby
D) said distal body ends (106) of said spacers (152) are oriented towards said distal end of said longitudinal member and said proximal body ends (104) of said spacers (152) are oriented towards said proximal end (134) of said longitudinal member.

2. The device of claim 1, wherein the spacer (152) has a cavity at the proximal body end (104) configured and dimensioned for receiving a distal body end of an adjacent spacer (152).

3. The device of claim 1, wherein the spacer (152) has a frustoconical portion.

4. The device of claim 1, wherein the fixation element is held to the distal body end by a friction fit.

5. The device of claim 1, wherein the spacer (152) includes a conical tip portion adjacent the distal body end and a shoulder is positioned adjacent the tip portion and extends radially outward therefrom for contacting the proximal body end (104) of an adjacent spacer (152) when such spacers (152) are in abutting relationship.

6. The device of claim 1, wherein the spacers (152) are stackable such that a plurality of spacers (152) are positionable in abutting relationship.

7. The device of claim 1, wherein the spacers (152) are axially alignable within the channel (138).

8. The device of claim 1, wherein the piston (142) includes a frustoconical tip at a distal end and the tip is configured and dimensioned to be received in the proximal body end (104) of the spacer (152).

9. The device of claim 8, further including a tab (144) extending radially outward from the piston (142), the tab configured to be movable by a human finger such that the piston (142) may be moved with respect to the longitudinal member.

10. The device of claim 9, further comprising a spring which engages a slot in the longitudinal member for locating the piston (142) at a plurality of preselected locations with respect to the longitudinal member.

11. The device of claim 1, wherein the channel (138) has a front opening at the distal end of the longitudinal member such that the spacers (152) can travel through the opening.

12. The device of claim 11, wherein the channel (138) further includes a back opening at the proximal end (134) of the longitudinal member and the spacer (152) can travel through the back opening.

13. The device of claim 1, wherein a handle (132) is connected to the proximal end (134) of the longitudinal member, the handle (132) configured and dimensioned to receive the hand of a user.

## Patentansprüche

1. Vorrichtung zum Befestigen von Fixationselementen am Knochen, die ein entlang einer Längsachse verlaufendes Längselement (136) aufweist, welches sich von einem an seinem proximalen Ende (134) befindlichen Griff (132) zu einem distalen Ende hin erstreckt, welches einen darin verlaufenden Kanal (138), sowie einen in Bezug auf den Kanal (138) zentral angeordneten Kolben (142) mit manuellem Vorschub aufweist, wobei die Vorrichtung weiterhin folgendes umfasst:
A) eine Mehrzahl von Abstandselementen (152), die innerhalb des Kanals (138) positionierbar sind und mittels des Kolbens (142) bezüglich des Kanals (138) axial verschiebbar sind, wobei
B) jedes Abstandselement (152) einen Körper umfasst, der sich von einem distalen Körperende (106) zu einem proximalen Körperende (104) hin erstreckt und
C) das distale Körperende (106) entsprechend angepasst ist, um zumindest einen Abschnitt eines Fixationselements aufzunehmen, wobei
D) die distalen Körperenden (106) der Abstandselemente (152) zu dem distalen Ende des Längselements hin ausgerichtet sind und die proximalen Körperenden (104) der Abstandselemente (152) zu dem proximalen Ende (134) des Längselements hin ausgerichtet sind.

2. Vorrichtung nach Anspruch 1, wobei das Abstandselement (152) an dem proximalen Körperende (104) einen Hohlraum aufweist, welcher entsprechend ausgelegt und dimensioniert ist, um ein distales Körperende eines angrenzenden Abstandselements (152) aufzunehmen.

3. Vorrichtung nach Anspruch 1, wobei das Abstandselement (152) einen kegelstumpfförmigen Abschnitt aufweist.

4. Vorrichtung nach Anspruch 1, wobei das Fixationselement mittels Reibpassung an dem distalen Körperende festgehalten wird.

5. Vorrichtung nach Anspruch 1, wobei das Abstandselement (152) angrenzend an das distale Körperende einen konischen Endabschnitt beinhaltet und wobei angrenzend an den Endabschnitt eine Schulter positioniert ist und sich davon radial nach aussen erstreckt, um mit dem proximalen Körperende (104) eines angrenzenden Abstandselements (152) in Kontakt zu treten, wenn diese Abstandselemente (152) sich in aneinandergrenzender Beziehung befinden.

6. Vorrichtung nach Anspruch 1, wobei die Abstandselemente (152) stapelbar sind, so dass eine Mehrzahl von Abstandselementen (152) in aneinandergrenzender Beziehung positionierbar ist.

7. Vorrichtung nach Anspruch 1, wobei die Abstandselemente (152) innerhalb des Kanals (138) axial ausrichtbar sind.

8. Vorrichtung nach Anspruch 1, wobei der Kolben (142) an einem distalen Ende ein kegelstumpfförmiges Endstück beinhaltet und das Endstück entsprechend ausgelegt und dimensioniert ist, um in dem proximalen Körperende (104) des Abstandselements (152) aufgenommen zu werden.

9. Vorrichtung nach Anspruch 8, welche weiterhin eine Nase (144) aufweist, die sich von dem Kolben (142) radial nach aussen erstreckt, wobei die Nase entsprechend ausgelegt ist, um durch einen menschlichen Finger verschiebbar zu sein, so dass der Kolben (142) in Bezug auf das Längselement verschoben werden kann.

10. Vorrichtung nach Anspruch 9, welche weiterhin eine Feder umfasst, die mit einem in dem Längselement ausgebildeten Schlitz in Eingriff tritt, um den Kolben (142) in einer Mehrzahl von vorgewählten Stellungen in Bezug auf das Längselement zu positionieren.

11. Vorrichtung nach Anspruch 1, wobei der Kanal (138) an dem distalen Ende des Längselements eine vordere Öffnung aufweist, so dass die Abstandselemente (152) durch die Öffnung hindurchbewegt werden können.

12. Vorrichtung nach Anspruch 11, wobei der Kanal (138) weiterhin an dem proximalen Ende (134) des Längselements eine hintere Öffnung aufweist und das Abstandselement (152) durch die hintere Öffnung hindurchbewegt werden kann.

13. Vorrichtung nach Anspruch 1, wobei ein Griff (132) mit dem proximalen Ende (134) des Längselements verbunden ist, wobei der Griff (132) entsprechend ausgelegt und dimensioniert ist, um die Hand eines Benutzers aufzunehmen.

## Revendications

1. Dispositif permettant de fixer des éléments de fixation sur de l'os, lequel présente un élément longitudinal (136) s'étendant le long d'un axe longitudinal, et ce depuis une poignée (132) située à son extrémité proximale (134) jusqu'à une extrémité distale, et comprenant un canal (138) s'étendant à l'intérieur de celui-ci ainsi qu'un piston (142) à avance manuelle disposé de manière centrale dans ledit canal (138), le dispositif comprenant en outre :
A) une pluralité d'éléments d'espacement (152) pouvant être positionnés à l'intérieur du canal (138) et pouvant être déplacés par rapport au canal (138) au moyen du piston (142) de manière axiale,
B) chaque élément d'espacement (152) comprenant un corps qui s'étend depuis une extrémité de corps distale (106) jusqu'à une extrémité de corps proximale (104) et
C) l'extrémité de corps distale (106) étant adaptée de manière à recevoir au moins une partie d'un élément de fixation,
D) les extrémités de corps distales (106) des éléments d'espacement (152) étant orientées vers l'extrémité distale de l'élément longitudinal et les extrémités de corps proximales (104) des éléments d'espacement (152) étant orientées vers l'extrémité proximale (134) de l'élément longitudinal.

2. Dispositif selon la revendication 1, l'élément d'espacement (152) présentant au niveau de l'extrémité de corps proximale (104) une cavité qui est conçue et dimensionnée de manière à recevoir une extrémité de corps distale d'un élément d'espacement (152) adjacent.

3. Dispositif selon la revendication 1, l'élément d'espacement (152) présentant une partie tronconique.

4. Dispositif selon la revendication 1, l'élément de fixation étant maintenu sur l'extrémité de corps distale par ajustement par frottement.

5. Dispositif selon la revendication 1, l'élément d'espacement (152) comportant un bout conique adjacent à l'extrémité de corps distale et un épaulement étant positionné de manière adjacente audit bout, ledit épaulement s'étendant radialement vers l'extérieur afin d'entrer en contact avec l'extrémité de corps proximale (104) d'un élément d'espacement (152) adjacent lorsque ces éléments d'espacement (152) se trouvent dans une relation de contiguïté.

6. Dispositif selon la revendication 1, les éléments d'espacement (152) étant superposables de telle sorte qu'il est possible de mettre dans une relation de contiguïté une pluralité d'éléments d'espacement (152).

7. Dispositif selon la revendication 1, les éléments d'espacement (152) pouvant être orientés de manière axiale à l'intérieur du canal (138).

8. Dispositif selon la revendication 1, le piston (142) comportant à une extrémité distale un bout tronconique et le bout tronconique étant conçu et dimensionné de manière à pouvoir être reçu dans l'extrémité de corps proximale (104) de l'élément d'espacement (152).

9. Dispositif selon la revendication 8, lequel présente en outre un nez (144) qui s'étend radialement vers l'extérieur à partir du piston (142), ledit nez étant conçu de manière à pouvoir être déplacé par le doigt d'un être humain de sorte qu'il est possible de faire coulisser le piston (142) par rapport à l'élément longitudinal.

10. Dispositif selon la revendication 9, lequel comprend en outre un ressort qui entre en prise avec une fente ménagée dans l'élément longitudinal afin de placer le piston (142) dans une pluralité de positions pré-sélectionnées par rapport à l'élément longitudinal.

11. Dispositif selon la revendication 1, le canal (138) présentant au niveau de l'extrémité distale de l'élément longitudinal une ouverture avant, de sorte qu'il est possible de faire passer les éléments d'espacement (152) à travers ladite ouverture.

12. Dispositif selon la revendication 11, le canal (138) présentant en outre au niveau de l'extrémité proximale (134) de l'élément longitudinal une ouverture arrière, de sorte qu'il est possible de faire passer l'élément d'espacement (152) à travers ladite ouverture arrière.

13. Dispositif selon la revendication 1, une poignée (132) étant reliée à l'extrémité proximale (134) de l'élément longitudinal, la poignée (132) étant conçue et dimensionnée de manière à recevoir la main d'un utilisateur.
